# EUROPEAN PATENT SPECIFICATION

(11) **EP 1 030 688 B1**
(45) Date of publication and mention of the grant of the patent: **29.09.2004**
(21) Application number: 98957511.3
(22) Date of filing: 03.11.1998
(51) Int. Cl.: A61K 47/18, A61K 38/28

(54) **METHOD FOR DERMALLY ADMINISTERING POLYPEPTIDES**
VERFAHREN ZUR DERMALEN VERABREICHUNG VON POLYPEPTIDEN
PROCEDE D'ADMINISTRATION DERMALE DE POLYPEPTIDES

(30) Priority: 12.11.1997 US 969217
(43) Date of publication of application: 30.08.2000
(73) Proprietor: ALZA CORPORATION, Palo Alto California 94303-0802 (US)
(72) Inventor: LEUNG, Iris, Ka, Man, Chesterfield, MO 63017-5514 (US)
(74) Representative: Campbell, Neil Boyd
(86) International application number: PCT/US1998/023298
(87) International publication number: WO 1999/024071

(56) References cited:
- EP-A- 0 692 489
- WO-A-92/12999
- WO-A-96/11705
- WO-A-97/39768
- US-A- 4 371 523
- US-A- 5 312 326
- US-A- 5 580 856
- LOUGHEED W.D. ET AL.,: "Insulin aggregation in artifical delivery systems" DIABETOLOGIA, vol. 19, no. 1, - July 1980 pages 1-9, XP002100222 cited in the application

## Description

### Technical Field

The invention relates generally to transdermal drug delivery. More particularly, the invention relates to a method for decreasing self-association of polypeptides to aid in the transdermal delivery thereof.

### Background of the Invention

Transdermal (i.e., through the skin) delivery of therapeutic agents affords a comfortable, convenient and noninvasive technique for administering drugs. The method provides several advantages over conventional modes of drug delivery. For example, variable rates of absorption and (e.g., hepatic) metabolism encountered in oral treatment are avoided, and other inherent inconveniences -- e.g., gastrointestinal irritation and the like -- are eliminated. Transdermal delivery also allows a high degree of control over blood concentrations of a particular drug and is an especially attractive administration route for drugs with narrow therapeutic indexes, short half-lives and potent activities.

Transdermal delivery can be either passive or active. Many drugs are not suitable for passive transdermal drug delivery because of their size, ionic charge characteristics and hydrophobicity. One method of overcoming this limitation is the use of low levels of electric current to actively transport drugs into the body through intact skin. This technique is known as "electrotransport" or "iontophoretic" drug delivery. The technique provides a more controllable process than passive transdermal drug delivery since the amplitude, timing and polarity of the applied electric current is easily regulated using standard electrical components. In this regard, electrotransport drug flux can be from 50% to several orders of magnitude greater than passive transdermal flux of the same drug.

Electrotransport devices generally employ at least two electrodes. Both of these electrodes are positioned in intimate electrical contact with some portion of the skin of the body. One electrode, called the active or donor electrode, is the electrode from which the therapeutic agent is delivered into the body. The other electrode, called the counter or return electrode, serves to close the electrical circuit through the body. In conjunction with the patient's skin, the circuit is completed by connection of the electrodes to a source of electrical energy, e.g., a battery, and usually to circuitry capable of controlling current passing through the device.

Depending upon the electrical charge of the species to be delivered transdermally, either the anode or cathode may be the active or donor electrode. Thus, if the ionic substance to be driven into the body is positively charged, the positive electrode (the anode) will be the active electrode and the negative electrode (the cathode) will serve as the counter electrode, completing the circuit. On the other hand, if the ionic substance to be delivered is negatively charged, the cathodic electrode will be the active electrode and the anodic electrode will be the counter electrode. Alternatively, both the anode and the cathode may be used to deliver drugs of appropriate charge into the body. In this case, both electrodes are considered to be active or donor electrodes. In other words, the anodic electrode can deliver positively charged agents into the body while the cathodic electrode can deliver negatively charged agents into the body.

Existing electrotransport devices additionally require a reservoir or source of the therapeutic agent that is to be delivered into the body. Such drug reservoirs are connected to the anode or the cathode of the electrotransport device to provide a fixed or renewable source of one or more desired species or agents. Examples of reservoirs and sources include a pouch as described in U.S. Patent No. 4,250,878 to Jacobsen; a pre-formed gel body as disclosed in U.S. Patent No. 4,382,529 to Webster; and a glass or plastic container holding a liquid solution of the drug, as disclosed in the figures of U.S. Patent No. 4,722,726 to Sanderson et al.

Of particular interest herein is the transdermal delivery of peptides, polypeptides, and proteins because of the problems encountered with more common drug administration routes such as oral delivery. Polypeptide and protein molecules are highly susceptible to degradation by proteolytic enzymes in the gastrointestinal tract and are subjected to an extensive hepatic metabolism when taken orally. Thus, these substances usually require parenteral administration to achieve therapeutic levels in the patient's blood. The most conventional parenteral administration techniques are hypodermic injections and intravenous administration. Polypeptides and proteins are, however, inherently short acting in their biological activity, requiring frequent injections, often several times a day, to maintain the therapeutically effective levels needed. Patients frequently find this treatment regimen to be inconvenient and painful. Such therapy also includes risk of, e.g., infection.

Much effort has been expended to find other routes (other than parenteral injections) for effective administration of pharmaceutical polypeptides and proteins. Administration routes with fewer side effects as well as better patient compliance have been of particular interest. Such alternative routes have generally included "shielded" oral administration wherein the polypeptide/protein is released from a capsule or other container after passing through the low pH environment of the stomach, delivery through the mucosal tissues, e.g., the mucosal tissues of the lung with inhalers or the nasal mucosal tissues with nasal sprays, and implantable pumps. Unfortunately, these alternative routes of polypeptide/protein delivery have met with only limited success.

A number of investigators have disclosed electrotransport delivery of polypeptides and proteins. An early study by R. Burnette et al. *J*. *Pharm. Sci.* (1986) 75:738, involved *in vitro* skin permeation of thyrotropin releasing hormone, a small tripeptide molecule. The electrotransport flux was found to be higher than passive diffusional flux. Chien et al. *J. Pharm*. *Sci.* (1988) 78:376, in both *in vitro* and *in vivo* studies, showed that transdermal delivery of vasopressin and insulin via electrotransport was possible. See, also, Maulding et al., U.S. Statutory Invention Registration No. H1160, which discloses electrotransport delivery of calcitonin in minipigs.

However, transdermal delivery of polypeptides and proteins has also encountered technical difficulties. For example, skin irritation can occur due to water hydrolysis at the interface between the electrode and the drug solution or electrolyte salt solution. The products of such hydrolysis, hydronium ions at the anode and hydroxyl ions at the cathode, compete with drug ions of like charge for delivery into the skin, altering skin pH and causing irritation. U.S. Patent No. 5,533,971, to Phipps et al., describes this problem in more detail and reports the use of amino acid buffers, including histidine buffers, for reducing skin irritation.

Additionally, certain polypeptides, particularly those that are not native to the animal being treated, may cause skin reactions, e.g., sensitization or irritation. Many polypeptides are also unstable and degrade rapidly. In this regard, WO97/39768 discloses a formulation stabilised against deamination and aggregation comprising growth hormone, an amino acid and a non-ionic detergent. Similarly EP-A-0909564 disdoses an erythropoietin solution containing an amino acid and having long term storage. In an alternative storage method, US 5,880,856 discloses dried proteins stabilised by a range of stabilisers. Also, International Publication No. WO 93/12812, published 8 July 1993, describes the use of histidine buffers to chemically stabilise growth hormone formulations. Furthermore, certain polypeptide drugs rapidly aggregate in aqueous solution which can cause both delivery and solubility problems.

For example, aqueous insulin, at concentrations relevant for pharmaceutical formulations, has a tendency to form dimers, which in turn self-associate into tetramers, hexamers, stacked hexamers and other polymeric species, with a concomitant decrease in solubility. These aggregates can obstruct mechanical parts of continuous delivery devices and are difficult, if not impossible, to deliver transdermally. This tendency is exacerbated by the presence of metal ions, such as zinc, traditionally used in insulin formulations to stabilise and prolong the activity of insulin.

Attempts have been made to decrease self-association of proteins such as insulin. For example, US 4,371,523 discloses a method of reducing aggregation of insulin in aqueous solutions comprising an organic compound having at least two carboxylic acid moieties and at least one amino, or amino derived, moiety. In addition, Ogiso et al., *Biol*. *Pharm. Bull.* (1996) 19:1049-1054, report the use of a Gly-HCl buffer to promote dissociation of porcine insulin oligomers prior to percutaneous absorption thereof. Bringer et al., *Diabetes* (1981) 30:83-85 report that the dicarboxylic amino acids, Asp and Glu, at their isoelectric pH, reduce aggregation of insulin in solution. The experimenters explain that acid pH (3.5) seems necessary in order for aggregation to be retarded using these amino acids. However, insulin is chemically unstable in acid. U.S. Patent No. 4,940,456, to Sibalis et al., describes insulin compositions for electrolytic transdermal transport which include urea, propylurea, potassium iodide, sodium perchlorate or guanidine hydrochloride as dissociating agents.

Insulin analogs have also been developed that reportedly have decreased tendencies toward self-association. For example, U.S. Patent No. 5,164,366, to Balschmidt et al., describes insulin analogs with deletions of certain amino acids, such as Phe^{B24} or Phe^{B25}. International Publication No. WO 92/12999, published 6 August 1992, describes human insulin analogs with selected amino acid residues substituted with Asp and Glu residues. EP Patent Publication No. 214,826 B1, published 18 March 1987, reports insulin analogs having amino acid substitutions, particularly in the B9-B12 region and the B26-B28 positions, wherein the residue substituted for the natural amino acid is more hydrophilic. Preferred amino acid substitutions include Asp, Glu, Ser, Thr, His and Ile. However, many of these analogs display reduced biological activity.

Thus, alternative methods for decreasing self-association of polypeptide drugs such as insulin, in the context of transdermal delivery. would be desirable.

### Disclosure of the invention

Accordingly, the present invention is based on a method for preventing self-association of insulin and other bioactive polypeptides while at the same time aiding in solubilisation of such molecules. The method uses histidine compounds and, by virtue of the decrease of self-association, allows for more efficient deliver of proteins transdermally, such as by electrotransport and passive transdermal delivery, in therapeutically effective amounts.

Accordingly, in one embodiment, the invention provides the use of a polypeptide and a histidine compound in an amount sufficient to decrease the tendency of said polypeptide to self-associate in the manufacture of a composition for delivering said polypeptide through the skin by electrotransport or passive transdermal delivery.

Preferably, the histidine compound is L-histidine or L-glycyl-histidine and the polypeptide is an insulin compound, with or without zinc, such as a zinc-free human insulin compound or a human Lys^{B28}Pro^{B29} insulin analog.

In another embodiment, the subject invention is directed to a use as hereinbefore defined in which the formation of hexamers and larger species of a human insulin compound is decreased. This comprises combining the insulin compound with a histidine compound at about pH 7 to about pH 8. The concentration of histidine is at least about 10 mmolar (mM).

In yet another embodiment, the invention provides a composition suitable for delivery through a body surface by electrotransport or passive transdermal delivery comprising an insulin compound and a histidine compound in an amount sufficient to decrease the tendency of said insulin compound to self-associate.

In another embodiment, the invention is directed to a use as hereinbefore defined for delivering a human insulin compound through a body surface by electrotransport. This comprises:
(a) providing a composition comprising the insulin compound and a histidine compound at about pH 7 to about pH 8, wherein the concentration of the histidine compound is about 10 mmolar to about 250 mmolar;
(b) delivering the composition through the body surface by electrotransport.

In yet another embodiment, the invention is directed to a use as hereinbefore defined for administering a human insulin compound through a body surface by passive transdermal delivery. This comprises:
(a) providing a composition comprising said insulin compound and a histidine compound, wherein said histidine compound is present in said composition in an amount sufficient to decrease the tendency of said insulin to self-associate; and
(b) administering said composition through the body surface by passive transdermal delivery.

These and other embodiments of the subject invention will readily occur to those of skill in the art in light of the disclosure herein.

### Brief Description of the Drawings

Figure 1 shows the effect of increasing histidine concentration on the solubility of wild-type human insulin with two zinc bound per hexamer at pH 7.5.
Figure 2 is a schematic view of a representative electrotransport drug delivery device which can be used with the present invention.
Figure 3 is a cross-sectional view of a representative passive transdermal drug delivery device which can be used with the present invention.
Figure 4 is a cross-sectional view of an alternate passive transdermal drug delivery device which can be used with the present invention.
Figure 5 is a graph depicting the average molecular weights of zinc-free Lys^{B28}Pro^{B29} human insulin as a function of insulin concentration for the 240 mM histidine data taken from Table III in the examples.
Figure 6 is a graph depicting the average molecular weights of zinc-free Lys^{B28}Pro^{B29} human insulin as a function of insulin concentration for the 0 mM histidine data taken from Table III in the examples.
Figure 7 is a graph depicting the average molecular weights of zinc-free wild-type human insulin as a function of insulin concentration for the 240 mM histidine data taken from Table lV in the examples.
Figure 8 is a graph depicting the average molecular weights of zinc-free wild-type human insulin as a function of insulin concentration for the 0 mM histidine data taken from Table IV in the examples.

### Detailed Description of the Invention

The practice of the present invention will employ, unless otherwise indicated, conventional methods of protein chemistry, electrochemistry and biochemistry within the skill of the art. Such techniques are explained fully in the literature. See, e.g., T.E. Creighton, *Proteins: Structures and Molecular Properties* (W.H. Freeman and Company, 1993); A.L. Lehninger, *Biochemistry* (Worth Publishers, Inc., 1975); J.S. Newman, *Electrochemical Systems* (Prentice Hall, 1973); and A.J. Bard and L.R. Faulkner, *Electrochemical Methods, Fundamentals and Applications* (John Wiley & Sons, 1980).

It must be noted that, as used in this specification and the appended claims, the singular forms "a", "an" and "the" include plural referents unless the content clearly dictates otherwise. Thus, for example, reference to "a polypeptide" includes a mixture of two or more polypeptides, and the like.

The following amino acid abbreviations are used throughout the text:

| | |
|---|---|
| Alanine: Ala (A) | Arginine: Arg (R) |
| Asparagine: Asn (N) | Aspartic acid: Asp (D) |
| Cysteine: Cys (C) | Glutamine: Gln (Q) |
| Glutamic acid: Glu (E) | Glycine: Gly (G) |
| Histidine: His (H) | Isoleucine: Ile (I) |
| Leucine: Leu (L) | Lysine: Lys (K) |
| Methionine: Met (M) | Phenylalanine: Phe (F) |
| Proline: Pro (P) | Serine: Ser (S) |
| Threonine: Thr (T) | Tryptophan: Trp (W) |
| Tyrosine: Tyr (Y) | Valine: Val (V) |

### I. Definitions

In describing the present invention, the following terms will be employed, and are intended to be defined as indicated below.

The terms "polypeptide," "polypeptide agent" and "polypeptide drug" are used interchangeably herein to denote any bioactive polymer of amino acid residues. The terms encompass peptides, oligopeptides, dimers, multimers, and the like. Such polypeptides can be derived from natural sources or can be synthesized or recombinantly produced. The terms also include postexpression modifications of the polypeptide, for example, glycosylation, acetylation, phosphorylation, etc.

A polypeptide drug or agent as defined herein is generally made up of one or more of the 20 natural amino acids, listed above and may also include any of the several known amino acid analogs, both naturally occurring and synthesized analogs, such as but not limited to homoisoleucine, 2-(methylenecyclopropyl)glycine, S-methylcysteine, S-(prop-I-enyl)cysteine, homoserine, ornithine, norleucine, norvaline, homoarginine, 3-(3-carboxyphenyl)alanine, cyclohexylalanine, mimosine, pipecolic acid, 4-methylglutamic acid, canavanine, 2,3-diaminopropionic acid, and the like. The polypeptide can also exist in neutral or salt forms, e.g., acid addition salts (formed with the free amino groups of the analog polypeptides) and which are formed with inorganic acids such as, for example, hydrochloric or phosphoric acids, or such organic acids as acetic, succinic, maleic, tartaric, mandelic, and the like. Salts formed from free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, and the like. Examples of polypeptide agents which will find use in the present invention are set forth below.

The term "insulin compound" as used herein refers to a compound having a molecular structure similar or identical to native insulin or proinsulin, including a molecule with similar or identical tertiary conformation to native insulin or proinsulin, and which retains insulin activity, i.e., the ability to regulate blood glucose levels. Such compounds may include amino acid additions, substitutions and deletions, relative to the native molecule, so long as the modifications do not destroy insulin activity. Examples of insulin compounds with amino acid substitutions relative to native insulin include Lys^{B28}Pro^{B29} human insulin and Asp^{B28} human insulin. Furthermore, for purposes of the present invention, an insulin compound may be derived from any mammalian source, such as human, bovine, canine, equine, ovine, porcine, cetacean, etc. The insulin compound may be purified directly from the pancreas of the source organism, or may be recombinantly or synthetically produced. See, e.g., Brange, J. *Galenics of Insulin, The* *Physico-chemical and Pharmaceutical Aspects of Insulin and Insulin Preparations* (Springer-Verlag) for various methods of obtaining insulin.

Additionally, the term "insulin compound" as used herein denotes an insulin compound with or without associated metals. In this regard, it has been found that metals, such as zinc and calcium, prolong the activity of insulin as well as increase physical stability of the molecule. Thus, an insulin compound for use in the present methods includes, without limitation, metal-free insulin, as well as insulin in association with an appropriate metal, including but not limited to insulin having from about 2 Zn²⁺ molecules/hexamer to about 4 Zn²⁺ molecules/hexamer. See, e.g., U.S. Patent No. 4,476,118, for a description of such compounds, as well as methods of making the same. Further examples of insulin compounds for use with the present invention are described more fully below.

The term "histidine compound" as used herein refers to the amino acid L-His, as well as amino acid analogs of L-His which retain the ability to decrease oligomer formation of a given polypeptide, as defined below. Such analogs include, without limitation, dipeptides and tripeptides which contain His, such as but not limited to, His-Gly, Gly-His, Ala-His, 3 methyl-His, 1 methyl-His, carnosine, His-Ser and His-Ala.

A histidine compound "decreases oligomer formation" of a given polypeptide when self-association of the polypeptide resulting in oligomers, such as tetramers, hexamers, stacked hexamers, and other polymers, is either retarded (e.g., oligomer formation is at least partially prevented), or reversed (e.g., already aggregated polypeptides are dissociated), by the presence of the histidine compound. The ability of a histidine compound to decrease oligomer formation can be determined by assessing the presence of oligomeric species in the presence and absence of the histidine compound in question. Such formation can be determined using analytical ultracentrifugation (see, e.g., *Modem Analytical Ultracentrifugation,* Schuster and Laue eds. 1994, Birkh@user; and *Analytical Ultracentrifugation* *in Biochemistry and Polymer Science,* Harding, Rowe and Horton eds., 1992, The Royal Society of Chemistry), such as sedimentation equilibrium studies as described in the examples, spectrophotometric determinations (see, e.g., Ogiso et al., *Biol*. *Pharm*. *Bull.* (1996) 19:1049-1054), osmometry, gel filtration, and the like. For a description of such methods, see, e.g., Valdes and Ackers, *Methods in Enzymology,* Vol. 61 (Enzyme Structure, part H, Hirs and Timasheff, eds.) Academic Press, 1979, pp. 125-142.

The term "passive transdermal delivery" refers to the delivery through a body surface (e.g., skin) of one or more pharmaceutically active polypeptide agents to be available for distribution via the systemic circulation, without the aid of an applied electromotive force. Passive transdermal delivery can be accomplished using a number of means including, without limitation, direct application to the skin, transdermal patches, membrane-moderated systems to provide controlled delivery, adhesive diffusion-controlled systems, matrix dispersion-type systems, and microreservoir systems. Such systems are known in the art and are discussed in detail in *Remington: The Science and Practice of Pharmacy,* Mack Publishing Company, Easton, Pennsylvania, 19th edition, 1995. Penetration enhancers can be used to facilitate absorption through the skin. Such penetration enhancers include solvents such as water, alcohols including methanol, ethanol, 2-propanol and the like, alkyl methyl sulfoxides, pyrrolidones, laurocapram, acetone, dimethylacetamide, dimethyl formamide, tetrahydrofurfuryl; surfactants; and chemicals such as urea, N,N-diethyl-m-toluamide, and the like.

The terms "electrotransport", "iontophoresis", and "iontophoretic" are used herein to refer to the delivery through a body surface (e.g., skin) of one or more pharmaceutically active polypeptide agents by means of an applied electromotive force to an agent-containing reservoir. The agent may be delivered by electromigration, electroporation, electroosmosis or any combination thereof. Electroosmosis has also been referred to as electrohydrokinesis, electro-convection, and electrically induced osmosis. In general, electroosmosis of a species into a tissue results from the migration of solvent in which the species is contained, as a result of the application of electromotive force to the therapeutic species reservoir, i.e., solvent flow induced by electromigration of other ionic species. During the electrotransport process, certain modifications or alterations of the skin may occur such as the formation of transiently existing pores in the skin, also referred to as "electroporation". Any electrically assisted transport of species enhanced by modifications or alterations to the body surface (e.g., formation of pores in the skin) are also included in the term "electrotransport" as used herein. Thus, as used herein, the terms "electrotransport", "iontophoresis" and "iontophoretic" refer to (1) the delivery of charged agents by electromigration, (2) the delivery of uncharged agents by the process of electroosmosis, (3) the delivery of charged or uncharged agents by electroporation, (4) the delivery of charged agents by the combined processes of electromigration and electroosmosis, and/or (5) the delivery of a mixture of charged and uncharged agents by the combined processes of electromigration and electroosmosis.

A polypeptide shows "enhanced electrotransport" when electrotransport flux of the polypeptide through the body surface (e.g., the skin or mucosa) is increased in the presence of a histidine compound, as compared to the flux in the absence of the histidine compound, as determined using standard methods of measurement. For example, transdermal electrotransport flux can be assessed using a number of *in vivo* or *in vitro* methods, well known in the art. *In vitro* methods include clamping a piece of skin of an appropriate animal (e.g., human cadaver skin) between the donor and receptor compartments of an electrotransport flux cell, with the stratum corneum side of the skin piece facing the donor compartment. A liquid solution or gel containing the drug to be delivered is placed in contact with the stratum corneum, and electric current is applied to electrodes, one electrode in each compartment. The transdermal flux is calculated by sampling the amount of drug in the receptor compartment. Two successful models used to optimize transdermal electrotransport drug delivery are the isolated pig skin flap model of Riviere, Heit et al, *J*. *Pharm*. *Sci.* (1993) 82:240-243, and the use of isolated hairless skin from hairless rodents or guinea pigs. See, Hadzija et al., *J*. *Pharm. Pharmacol*. (1992) 44:387-390. See, also, Ogiso et al., *Biol*. *Pharm. Bull.* (1996) 19:1049-1054, for a description of a method for evaluating percutaneous absorption of insulin.

### II. Modes of Carrying Out the Invention

The present invention concerns the use of histidine compounds to decrease self-association of a polypeptide molecule, thereby enhancing transdermal delivery of the polypeptide molecule as compared to the delivery of the untreated polypeptide. The method therefore permits increased efficiency of the transdermal delivery of a large number of substances, and allows for the transdermal delivery of molecules that would not otherwise be amenable to such delivery. Additionally, the method increases the solubility of the polypeptide agent so treated and decreases the potential for immunological reactions that might occur against aggregates of otherwise endogenous substances.

The present invention will find use with a wide variety of proteins and polypeptide agents that have the tendency to aggregate, such as a number of polypeptides derived from eucaryotic, procaryotic and viral sources, as well as synthetic peptides. Such polypeptides include without limitation, peptide drugs which are antibiotics and antiviral agents, antineoplastics, immunomodulators, peptide hormones such as insulin, proinsulin, growth hormone, GHRH, LHRH, EGF, Somatostatin, SNX-111, BNP, insulinotropin, ANP, and glycoprotein hormones such as, FSH, LH, PSH and hCG.

The present invention has been exemplified using insulin and insulin analogs but is not limited to insulin compounds. Insulin was chosen to illustrate the invention based on its tendency to self-associate into hexameric and polymeric structures termed "stacked hexamers." Such association inhibits transdermal delivery of the polypeptide and can cause irritation at the delivery site.

Examples of insulin compounds for use with the present methods include any commercially available insulins, such as, for example, recombinant human insulin from Sigma, St. Louis, MO, formulated as neutral solutions or suspensions of zinc insulin. Such preparations of insulin contain a minimum of two zinc ions bound per hexamer and have an insulin concentration from about 0.2 to about 3.0 mM (1 mg mL⁻¹ to 18 mg mL⁻¹). However, insulin preparations including higher concentrations of insulin, up to about 17 mM insulin will also find use herein. Insulin devoid of metals such as zinc can also be used with the present methods and the concentration can range from about 0.1 to 30 mM. Insulin analogs for use as the insulin compound herein include commercially available human insulin analogs such as a Lys^{B28} and Pro^{B29} insulin, available from Lilly (Indianapolis, IN) as Humalog® insulin lispro injection, described further in the examples; insulin compounds containing protamine, such as NPH (Neutral Protamine Hagedorn) and isophane insulin (available from various manufacturers); and Lente and Biphasic insulins (available from various manufacturers). See, e.g., *Remington: The Science and Practice of Pharmacy*, Mack Publishing Company, Easton, Pennsylvania, 19th edition, 1995, for a description of these and other insulin compounds.

Other insulin analogs for use herein include, but are not limited to, analogs such as those described by Marki et al., Z. *Physiol*. *Chem*. (1979) 360:1619-1632, substituted at amino acid positions 2, 5, 6, 7, 8 and 11 of the A-chain and 5, 7, 13 and 16, of the B-chain; sulphated insulins such as those described by Albisser et al., in U.S. Pharmacopeial Convention, Rockville, MD. (Gueriguian et al., eds.) pp. 84-95; Des-Phe insulin (having the N-terminal amino acid of the B-chain deleted); insulin analogs with additional deletions of certain amino acids, such as deletion of Phe^{B24} or Phe^{B25} (U.S. Patent No. 5,164,366, to Balschmidt et al.); insulin analogs having amino acid substitutions, particularly in the B9-B12 region and the B26-B28 positions, wherein the residue substituted for the natural amino acid is more hydrophilic and is generally Asp, Glu, Ser, Thr, His and Ile (EP Patent Publication No. 214,826 B1, published 18 March 1987); human insulin analogs with selected amino acid residues substituted with Asp and Glu residues (International Publication No. WO 92/12999, published 6 August 1992), and the like.

Histidine compounds for use with the present invention include L-His, and analogs thereof, such as but not limited to, dipeptides and tripeptides which contain His, such as His-Gly, Gly-His, Ala-His, 3 methyl-His, 1 methyl-His, L-carnosine (also known as β-Ala-His), His-Ser and His-Ala. The choice of an appropriate histidine compound is within the skill in the art and will be determined based largely on the particular polypeptide in question.

The histidine compound will generally be present at its isoelectric point and in a concentration of from about 1 mM to 330 mM, more preferably about 10 mM to about 250 mM, and most preferably about 25 mM to about 250 mM. The optimal histidine concentration is dependent on a number of factors including insulin concentration, concentration of other salts (e.g., NaCl), the presence or absence of zinc, the presence or absence of preservatives, the tendency of the polypeptide to form oligomers, and the like. In general, the concentration of histidine is at least about 10 mM. Those skilled in the art of protein formulations can easily determine the optimal histidine concentration for the particular variables (e.g., insulin concentration, salt concentration, presence or absence of zinc, preservative or no preservative) used in a particular application or formulation.

The polypeptide will be present in a therapeutically effective amount, that is, an amount sufficient to achieve the desired therapeutic result. The exact amount required will vary from subject to subject, depending on the species, age, and general condition of the subject, the severity of the condition being treated, and the particular polypeptide drug of interest. Therapeutically effective doses are easily determined by one of skill in the art using e.g., standard dose response curves and the like. For example, if the polypeptide is insulin, it will normally be present in a concentration from about 0.1 to about 30 mM, more preferably 0.2 to about 20 mM and most preferably about 0.3 to about 17 mM, the concentration depending on the particular insulin compound used and whether the molecule includes bound zinc.

When L-His is used along with a commercially available human insulin, which generally includes insulin in the form of hexamers and stacked hexamers, insulin will usually be present in a concentration of about 0.2 mM to about 17 mM (1 mg mL⁻¹ to 100 mg mL⁻¹) and L-His present in a concentration of about 25-250 mM. One of skill in the art can readily determine the appropriate amount of insulin and L-His for use in the method of the invention.

Polypeptide drugs for use in the present invention may be negatively charged, positively charged, or neutral, the choice of which will depend on, among other factors, the particular histidine compound used, as well as the desired pH. Determination of these parameters is well within the skill in the art. For example, when L-His is used as the histidine compound and the pH of the composition is 7-8, the insulin compound will be negatively charged.

Generally, the pH of the final solution will be from about pH 6 to about pH 8.5, more preferably pH 7 to about pH 8. However, the pH of the solution can vary depending again on the particular polypeptide and histidine compound used in the method.

The polypeptide and histidine compounds are generally present in pharmaceutically acceptable excipients such as water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, preservatives, pH buffering agents and the like, for example, sodium acetate, sorbitan monolaurate, triethanolamine sodium acetate, triethanolamine oleate, etc. The choice of an appropriate excipient and additives is determined largely by the polypeptide and histidine compounds being used. For a discussion of polypeptide formulations, see, e.g., *Remington: The Science and Practice of Pharmacy,* Mack Publishing Company, Easton, Pennsylvania, 19th edition, 1995.

For insulin formulations, such substances include, without limitation, preservatives such as methylparaben and phenol (*m*-cresol); isotonic agents such as glycerol or salts, including but not limited to NaCl (generally at a concentration of about 1 to about 100 mM NaCl); and other additives and buffering agents such as sodium acetate, NaPO₄, and the like. For a discussion of insulin formulations, see, e.g., Brange, J., *Stability of Insulin* (Kluwer Academic Publishers); Brange, J. *Galenics of Insulin, The Physico-chemical and Pharmaceutical Aspects of Insulin and Insulin Preparations* (Springer-Verlag); and *Remington: The Science and Practice of Pharmacy*, Mack Publishing Company, Easton, Pennsylvania, 19th edition, 1995.

Once the desired polypeptide formulation with histidine is prepared, it can be delivered to the subject using any of several transdermal drug delivery systems and delivery is not limited to the use of one particular system. Examples of electrotransport drug delivery systems are described in, e.g., U.S. Patent Nos. 5,312,326 to Myers et al., 5,080,646 to Theeuwes et al., 5,387,189 to Gyory et al., and 5,169,383 to Gyory et al., the disclosures of which are incorporated by reference herein.

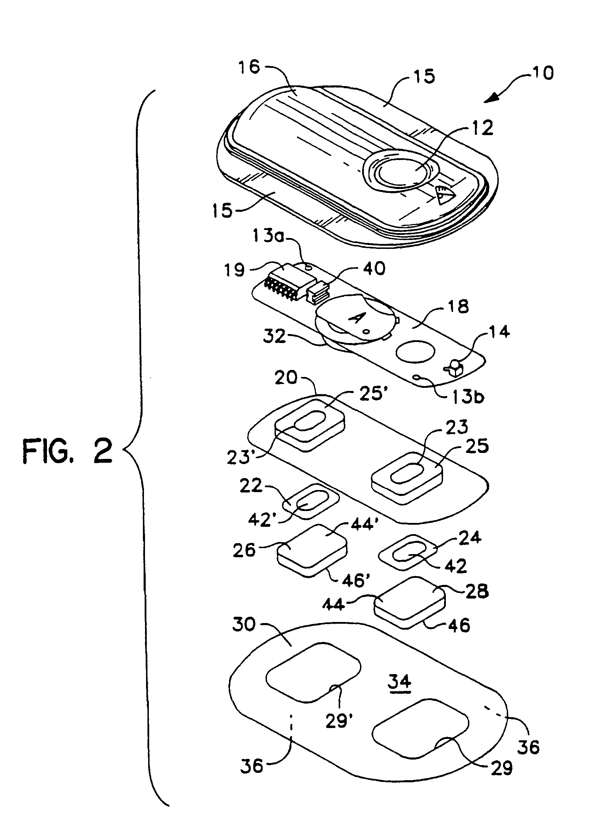

Figure 2 illustrates a representative electrotransport delivery device that may be used in conjunction with the present method. Device **10** comprises an upper housing **16,** a circuit board assembly **18**, a lower housing **20**, anode electrode **22**, cathode electrode **24,** anode reservoir **26,** cathode reservoir **28** and skin-compatible adhesive **30**. Upper housing **16** has lateral wings **15** which assist in holding device **10** on a patient's skin. Upper housing **16** is preferably composed of an injection moldable elastomer (e.g., ethylene vinyl acetate). Printed circuit board assembly **18** comprises an integrated circuit **19** coupled to discrete components **40** and battery **32**. Circuit board assembly **18** is attached to housing **16** by posts (not shown in Figure 2) passing through openings **13a** and **13b**, the ends of the posts being heated/melted in order to heat stake the circuit board assembly **18** to the housing **16**. Lower housing **20** is attached to the upper housing **16** by means of adhesive **30**, the upper surface **34** of adhesive **30** being adhered to both lower housing **20** and upper housing **16** including the bottom surfaces of wings **15**.

Shown (partially) on the underside of circuit board assembly **18** is a button cell battery **32**. Other types of batteries may also be employed to power device **10**.

The device **10** is generally comprised of battery **32**, electronic circuitry **19,40**, electrodes **22,24**, and drug/chemical reservoirs **26,28**, all of which are integrated into a self-contained unit. The outputs (not shown in Figure 2) of the circuit board assembly **18** make electrical contact with the electrodes **24** and **22** through openings **23,23'** in the depressions **25,25'** formed in lower housing **20**, by means of electrically conductive adhesive strips **42,42'**. Electrodes **22** and **24,** in turn, are in direct mechanical and electrical contact with the top sides **44',44** of drug reservoirs **26** and **28.** The bottom sides **46',46** of drug reservoirs **26,28** contact the patient's skin through the openings **29',29** in adhesive **30**.

Device **10** optionally has a feature which allows the patient to self-administer a dose of drug by electrotransport. Upon depression of push button switch **12**, the electronic circuitry on circuit board assembly **18** delivers a predetermined DC current to the electrodes/reservoirs **22,26** and **24,28** for a delivery interval of predetermined length. The push button switch **12** is conveniently located on the top side of device **10** and is easily actuated through clothing. A double press of the push button switch **12** within a short time period, e.g., three seconds, is preferably used to activate the device for delivery of drug, thereby minimizing the likelihood of inadvertent actuation of the device **10**. Preferably, the device transmits to the user a visual and/or audible confirmation of the onset of the drug delivery interval by means of LED **14** becoming lit and/or an audible sound signal from, e.g., a "beeper". Drug is delivered through the patient's skin by electrotransport, e.g., on the arm, over the predetermined delivery interval.

Anodic electrode **22** is preferably comprised of silver and cathodic electrode **24** is preferably comprised of silver chloride. Both reservoirs **26** and **28** are preferably comprised of polymer hydrogel materials. Electrodes **22,24** and reservoirs **26,28** are retained within the depressions **25',25** in lower housing **20**.

The push button switch **12**, the electronic circuitry on circuit board assembly **18** and the battery **32** are adhesively "sealed" between upper housing **16** and lower housing **20.** Upper housing **16** is preferably composed of rubber or other elastomeric material. Lower housing **20** is preferably composed of a plastic or elastomeric sheet material (e.g., polyethylene) which can be easily molded to form depressions **25,25'** and cut to form openings **23,23'**. The assembled device **10** is preferably water resistant (i.e., splash proof) and is most preferably waterproof. The system has a low profile that easily conforms to the body, thereby allowing freedom of movement at, and around, the wearing site. The reservoirs **26** and **28** are located on the skin-contacting side of the device **10** and are sufficiently separated to prevent accidental electrical shorting during normal handling and use.

The device **10** adheres to the patient's body surface (e.g., skin) by means of a peripheral adhesive **30** which has upper side **34** and body-contacting side **36**. The adhesive side **36** has adhesive properties which assures that the device **10** remains in place on the body during normal user activity, and yet permits reasonable removal after the predetermined (e.g., 24-hour) wear period. Upper adhesive side **34** adheres to lower housing **20** and retains lower housing **20** attached to upper housing 16.

The reservoirs **26** and **28** generally comprise a gel matrix, with the drug solution uniformly dispersed in at least one of the reservoirs **26** and **28.** Drug concentrations in the range of approximately 1 x 10⁻⁴ M to 1.0 M or more can be used, with drug concentrations in the lower portion of the range being preferred. Suitable polymers for the gel matrix may comprise essentially any nonionic synthetic and/or naturally occurring polymeric materials. A polar nature is preferred when the active agent is polar and/or capable of ionization, so as to enhance agent solubility. Optionally, the gel matrix will be water swellable. Examples of suitable synthetic polymers include, but are not limited to, poly(acrylamide), poly(2-hydroxyethyl acrylate), poly(2-hydroxypropyl acrylate), poly(N-vinyl-2-pyrrolidone), poly(n-methylol acrylamide), poly(diacetone acrylamide), poly(2-hydroxylethyl methacrylate), poly(vinyl alcohol) and poly(allyl alcohol). Hydroxyl functional condensation polymers (i.e., polyesters, polycarbonates, polyurethanes) are also examples of suitable polar synthetic polymers. Polar naturally occurring polymers (or derivatives thereof) suitable for use as the gel matrix are exemplified by cellulose ethers, methyl cellulose ethers, cellulose and hydroxylated cellulose, methyl cellulose and hydroxylated methyl cellulose, gums such as guar, locust, karaya, xanthan, gelatin, and derivatives thereof. Ionic polymers can also be used for the matrix provided that the available counterions are either drug ions or other ions that are oppositely charged relative to the active agent.

Thus, the polypeptide/histidine formulations used in the present invention will be incorporated into the drug reservoir, e.g., a gel matrix as just described, and administered to a patient using an electrotransport drug delivery system, optionally as exemplified hereinabove. Incorporation of the drug solution can be done any number of ways, i.e., by imbibing the solution into the reservoir matrix, by admixing the drug solution with the matrix material prior to hydrogel formation, or the like.

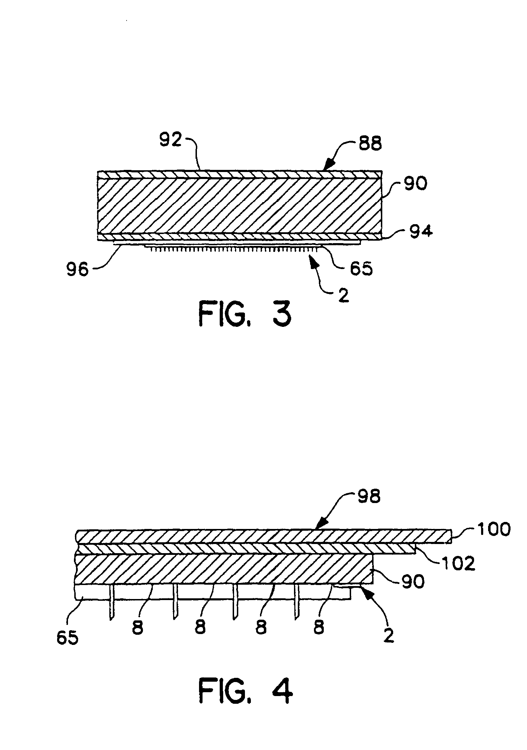

In other embodiments of the present invention, passive transdermal delivery can be used to administer the polypeptide/histidine formulations. It will be appreciated by those working in the field that the present invention can be used in conjunction with a wide variety of passive transdermal systems, as the invention is not limited in this regard. For examples of passive systems, reference may be had to, but not limited to, U.S. Patent Nos. 4,379,454 to Campbell et al., 4,588,580 to Gale et al., 4,832,953 to Campbell et al., 4,698,062 to Gale et al., 4,867,982 to Campbell et al., and 5,268,209 to Hunt at al., of which any of the disclosed systems can be used with the present invention. Two examples of passive transdermal delivery devices are illustrated in Figures 3 and 4.

In Figure 3, passive transdermal delivery device **88** comprises a reservoir **90** containing the formulation to be delivered transdermally. Reservoir **90** is preferably in the form of a matrix containing the formulation dispersed therein. Reservoir **90** is sandwiched between a backing layer **92,** which is impermeable to the agent, and an optional rate-controlling membrane **94**. In Figure 3, the reservoir **90** is formed of a material, such as a polymer, that is sufficiently viscous to maintain its shape. If a lower viscosity material is used for reservoir **90**, such as an aqueous gel, backing layer **92** and rate-controlling membrane **94** would be sealed together about their periphery to prevent leakage. Located below membrane **94** is skin piercing device **2** with connecting medium **65** on a skin facing surface thereof which extends through the openings (not shown) in device **2** to contact membrane **94**. The device **88** adheres to a body surface by means of contact adhesive layer **96** around the periphery of the device **2** and, optionally, by the anchoring elements of any of the embodiments described previously. In most instances, the connecting medium **65** will initially contain agent. A strippable release liner (not shown) is normally provided along the exposed surface of adhesive layer **96** and is removed prior'to application of device **10** to the body surface.

Alternatively, as shown in enlarged Figure 4, transdermal therapeutic device **98** may be attached to a body surface by means of a flexible adhesive overlay **100.** Device **98** is comprised of an agent-containing reservoir **90** which is preferably in the form of a matrix containing the agent dispersed therein. Connecting medium **65** extends through the openings **8** to contact the reservoir **90**. Alternatively, the matrix in reservoir **90** can extend through the openings **8** initially to be in contact with the connecting medium **65** or the reservoir and connecting medium can be the same. An impermeable backing layer **102** is provided adjacent one surface of reservoir **90**. Adhesive overlay **100** maintains the device on the body surface. Adhesive overly **100** can be fabricated together with, or provided separately from, the remaining elements of the device **98**. With certain formulations, the adhesive overlay **100** may be preferable to the contact adhesive **96** shown in Figure 3. This is true, for example, where the agent reservoir contains a material (such as, for example, an oily surfactant) which adversely affects the adhesive properties of the contact adhesive layer **96**. Impermeable backing layer **102** is preferably slightly larger than reservoir **90**, and in this manner prevents the agents in reservoir **90** from adversely interacting with the adhesive in overlay **100**. Optionally, a rate-controlling membrane (not shown in Figure 4) similar to membrane **94** in Figure 3 can be provided on the body surface side of reservoir **90**. A strippable release liner (not shown) is also normally provided with device **98** and is removed just prior to application of device **98** to the body surface.

The formulation of reservoir **90** may be aqueous or nonaqueous based. The formulation is designed to deliver the agent at the necessary fluxes. Aqueous formulations typically comprise water and about 1 to 60 weight percent of a hydrophilic polymer as a gelling agent, such as hydroxyethylcellulose, hydroxypropylcellulose, hydroxyethylmethacrylate and polymers used in soft contact lenses. Typical non-aqueous formulations are comprised of silicone fluid, silicone rubbers, hydrocarbon polymers, polyisobutylene, rubbers, or mineral oil. Mineral oil-based gels also typically contain 1 to 2 weight percent of a gelling agent such as colloidal silicon dioxide.

The reservoir matrix having agent therein should be compatible with the delivered agent, uptake inhibiting agent (if any) and any carrier therefore. When using an aqueous-based system, the reservoir matrix is preferably a hydrophilic polymer (e.g., a hydrogel). When using a non-aqueous-based system, the reservoir matrix is preferably composed of a hydrophobic polymer. Suitable polymeric matrices are well known in the transdermal drug delivery art.

When a constant agent delivery rate is desired, the agent is present in the matrix or carrier at a concentration in excess of saturation, the amount of excess being a function of the desired length of the agent delivery period of the system. The agent may, however, be present at a level below saturation as long as the polypeptide/histidine formulation and the uptake-inhibiting agent (if any) are continuously and co-extensively administered to the same body surface site in an amount and for a period of time sufficient to reduce or eliminate skin irritation by the agent.

In addition to the agent, the connecting medium may also contain dyes, pigments, inert fillers, permeation enhancers, excipients tackifiers, neutral polymers, surfactants, reagents, buffers, plasticizers, and other conventional components of pharmaceutical products or transdermal devices known in the art.

The amount of agent present in the reservoir and the size of the reservoir is generally non-limited and is an amount equal to or larger than the amount of agent that in its released form is effective in bringing about the desired local and/or systemic physiological and/or pharmacological effects.

The preferred form in which an agent is delivered generally determines the type of delivery system to be used, and vice versa. That is, the selection of a passive system which delivers the agent by diffusion or an electrically powered system which delivers the agent by electrotransport will be mostly determined by the form of the agent. For example, with passive delivery systems, it has generally been recognized that the agent is preferably delivered in either its free base or acid form, rather than in the form of a water soluble salt when the agent diffuses through the stratum corneum. On the other hand, with electrotransport delivery devices, it has been recognized that the agents should generally be soluble in water. It is generally believed that the pathways for passive and electrotransported transdermal agent delivery through intact skin are different, with passive delivery occurring through lipid regions (i.e., hydrophobic regions) of the skin and electrotransport delivery occurring through hydrophilic pathways or pores such as those associated with hair follicles and sweat glands. For the case of pierced skin, substantial passive flux through the created pathways which are aqueous can be expected. The agent for passive delivery in the case of pierced skin is generally hydrophilic (e.g., water soluble salt form) and the preferred form of an agent for electrotransport delivery is also hydrophilic (e.g., water soluble salt form). For passive delivery, a combination of ionized agent (e.g., water soluble) and unionized agent (e.g., hydrophilic) can be used.

In one preferred embodiment for passive transdermal delivery of insulin, the formulation will contain a histidine buffer and an insulin compound that is zinc-free and devoid of preservatives such as m-cresol or phenol, and either wild-type human insulin or an analog of insulin with a reduced tendency to self-associate, such as a human Lys^{B28}Pro^{B29} insulin analog. Such a formulation maximizes the proportion of the insulin molecules present as the more rapidly diffusing lower molecular weight species.

The polypeptide/histidine formulations may also be delivered using osmotic and pressure driven systems which deliver agents by connective flow carried by a solvent. In such systems, the agent preferably has sufficient solubility in the carrier solvent. It will be appreciated by those working in the field that the present invention can be used in conjunction with a wide variety of osmotic and pressure driven systems, as the invention is not limited to a particular device in this regard. For examples of osmotic and pressure driven devices, reference may be had to U.S. Patent Nos. 4,340,480 to Eckenhoff, 4,655,766 to Theeuwes et al., 4,753,651 to Eckenhoff, 5,279,544 to Gross et al., 4,655,766 to Theeuwes, 5,242,406 to Gross et al., and 4,753,651 to Eckenhoff any of which can be used with the present invention.

### III. Experimental

### Materials

Human insulin (produced by expression in *E*. *coli*), β-lactoglobulin, L-histidine (base) and serinamide were purchased from Sigma (St.Louis, MO). The Sigma insulin preparation contained about 0.4% Zn which was equivalent to about 2 zinc atoms per insulin hexamer. Humalog® (a Lys^{B28}Pro^{B29} human insulin analog) as well as Humulin® (human insulin injection), both of recombinant DNA origin and manufactured by Lilly (Indianapolis, IN) were purchased from commercial pharmacies. L-histidine (base) was obtained from J.T. Baker (Phillipsburg, NJ), as well as from Sigma (St. Louis, MO). Glacial acetic acid was obtained from J.T. Baker (Phillipsburg, NJ). Hydrochloric acid was purchased from Mallinckrodt (Paris, KY). Sodium chloride (NaCl) was supplied by Aldrich (St. Louis, MO). Lysozyme was obtained from Worthington Biochemical Corp. (Freehold NJ). L-Glycyl-L-histidine dipeptide was synthesized by Bachem Bioscience Inc. (King of Prussia, PA).

### Methods

### Preparation of zinc-free human insulins

All human insulins available from commercial sources contain about 2 bound zinc molecules per insulin hexamer. The zinc bound to the wild-type human insulin (available from Sigma or as Humulin® R) and the Lys^{B28}Pro^{B29} analog (available as Humalog®) can be removed by extensive dialysis against 10 mM acetic acid at 4°C. In the case of Humulin® R and Humalog®, the pH of the injectable insulin was first adjusted from neutral pH to pH 3.5 using glacial acetic and 1 N hydrochloric acid prior to dialysis. The insulin was then freeze-dried after dialysis. Zinc analysis of the freeze-dried material indicated that residual zinc was less than 0.03 zinc/hexamer.

### Preparation of L-histidine and L-glycyl-L-histidine buffers

Milli-Q water (Millipore, Medford, MA) was used for the preparation of all buffers. Buffers were filtered through 0.22 µm cellulose acetate membranes prior to use. The pH of a 252 mM L-histidine solution was about pH 7.62 ± 0.1 at 22°C and the pH of a 1 M L-glcyl-L-histidine buffer was about pH 7.68 ± 0.1 at 22°C.

### Preparation of serinamide buffer

A 100 mM serinamide buffer stock was prepared and the pH adjusted to 7.5 ± 0.1.

### Preparation of insulin in L-histidine and L-glycyl-L-histidine buffers

An insulin stock solution was prepared for each experiment performed in the analytical ultracentrifuge. The concentration of the stock solution was determined by diluting an aliquot into 6 M guanidine hydrochloride (Pierce, Rockford, IL) and monitoring its absorbance in a spectrophotometer (Aviv, model 14DS, Lakewood, NJ). The absorbance of the sample was corrected for light scattering prior to determining its concentration using a molar extinction coefficient of 1.109 mL mg⁻¹cm⁻¹ at 276 nm). For preparations that contained histidine, the insulin stock solution was made in either 250 or 330 mM L-histidine. For L-glycyl-L-histidine, the insulin stock solution was prepared in 260 mM and/or 1 M L-glycyl-L-histidine buffer. Typically, with wild-type insulin containing about 2 zinc/hexamer, a stock solution of up to 10 mM (about 60 mg mL⁻¹) was prepared in either L-histidine or L-glycyl-L-histidine. For preparations without histidine, a stock solution containing 7.1 mg mL⁻¹ of 2 zinc/hexamer wild-type insulin was prepared in 10 mM NaCl, adjusted to pH 7.5 with NaOH. In the case of zinc-free wild-type and/or Lys^{B28}Pro^{B29} human insulin analog, a stock solution of up to 17 mM (about 100 mg mL⁻¹) can be prepared in either 0.1 M NaCl, pH 7.5 and/or 250 mM histidine, 0.1 M NaCl, pH 7.5. The pH of the insulin stock solution was about 7.68 ± 0.1 in a 1 M glycyl-histidine buffer and pH 7.62 ± 0.1 in 250 mM histidine at 22°C.

Prior to the analytical ultracentrifuge run, the insulin stock solution was diluted with either histidine or glycyl-histidine buffer with or without the appropriate amount of NaCl such that the concentration of insulin varied from as low as 2 mg mL⁻¹ (0.35 mM ) to as high as 40 mg mL⁻¹ (7 mM). The final concentration of the L-histidine buffer into which the insulin was dissolved varied from 10 mM to 252 mM. For L-glycyl-L-histidine, wild-type insulin containing 2 zinc/hexamer preparations in 250 and 750 mM buffer were examined in the ultracentrifuge. In addition, sodium chloride, at a final concentration of 50 and 100 mM, was added to some of the insulin samples in L-histidine or L-glycyl-L-histidine buffers.

### Preparation of lysozyme & β-lactoglobulin

Lysozyme solutions (15 mg mL⁻¹) were prepared in 0.15 M NaCl with 0, 100, or 250 mM L-histidine. The pH of the histidine containing solutions was 7.60 ± 0.1 at 21°C; the pH of the lysozyme solution without L-histidine was adjusted with dilute base to be the same. Similarly the pH of β-lactoglobulin in 0.15 M NaCl was adjusted with dilute base to be the same pH as that in 250 mM L-histidine , pH 7.74 ± 0.1.

### Sedimentation equilibrium studies in the analytical ultracentrifuge

Sedimentation equilibrium experiments with various insulin formulations were performed at 32°C using an analytical ultracentrifuge (model XL-A or XL-I; Beckman, Palo Alto, CA).

Data at sedimentation equilibrium were obtained for all the samples using Rayleigh interference optics and/or scanning UV/Visible optics at various rotor speeds. For the latter, absorbance scans for insulin samples were monitored at several wavelengths, e.g. 248, 288, 291 and 295 nm. The molar extinction coefficient values were estimated from scans obtained at the start of the run with the monochromator set at the above wavelengths. These values were used to compute the molar association equilibrium constants of insulin under various conditions. Each data point in the absorbance scans was recorded as the average of ten scans with a radial distance increment of 0.002 cm. For the interference optical system, light at 675 nm was used to obtain sedimentation equilibrium data for insulin as well as lysozyme samples. The fringe displacement of a 1 mg mL⁻¹ polypeptide solution in a 1 cm path was taken as 2.77 fringes (McMeekin et al., *Biochem. Biophys. Res. Comm.* (1962) 7:151-156; Doty and Geiduschek, pp. 393-460, in *The Proteins, 1A*, edited by Neurath and Bailey, Academic Press, N. Y. (1943); Perlmann and Longsworth, *J*. *Amer. Chem*. *Soc.* (1948) 70: 2719-2724). This value was used to compute the molar association constants for insulin and lysozyme samples. The monomer molecular weight of human insulin was taken to be 5796 g mol⁻¹ with a partial specific volume of 0.727 mL g⁻¹ calculated from amino acid composition using Cohn and Edsall values for the residue partial specific volume. In the case of lysozyme, the molecular weight of the monomer was taken to be 14,315 g mol⁻¹ and a value of 0.703 mL g⁻¹ for the partial specific volume (Sophianopoulos et al., *J*. *Biol*. *Chem.* (1962) 237:1107). For β-lactoglobulin, UV absorbance scans were obtained at 280 nm and the molecular weight of the monomer was taken to be 18,400 g mol⁻¹. The partial volume for the latter is 0.747 mL g⁻¹ (Kelly and Reithel, *Biochemistry* (1971) 10:2639-2644) and the extinction coefficient used to compute molar association constants was 0.97 mL g⁻¹ cm⁻¹ (Wetlaufer and Lovrien, *J*. *Biol*. *Chem*. (1964) 243:596). The effective buoyant molecular weight of all proteins in the presence of L-histidine was computed using the excluded volume model (Jacobsen, et al., *Biochemistry* (1996) 35:13173-13179) in which the B_{AM} value was taken to be simply the monomer molecular weight multiply by the partial specific volume. The partial specific volume of L-histidine and L-glycyl-histidine was taken to be 0.641 mL g⁻¹ (pp. 370-381, *Proteins, Amino Acids, and Peptides,* (1943) edited by Cohn and Edsall, Hafner Publishing, N.Y.).

After centrifugation, the data obtained from the interference and absorbance scans were analyzed using known methods which included an algorithm based on equation 9 in Shire, et al., *Biochemistry* (1991) 30:7703-7711. The algorithm used included a modification in which the fitting parameter was (BM₁)^{½} instead of (B)^{½}. This analysis yields estimates for the association constants for user-specified model. The model can be that of an ideal monomer (the simplest model) or a monomer existing in chemical equilibrium with one, two, three or more aggregates of specific sizes. The most probable model is that which minimizes the sum of squares of the difference between the experimental absorbance from the theoretical absorbance, i.e. the model that has the lowest value for the root mean squared residuals.

### Example 1

### Effect of L-histidine on the solubility limit of insulin

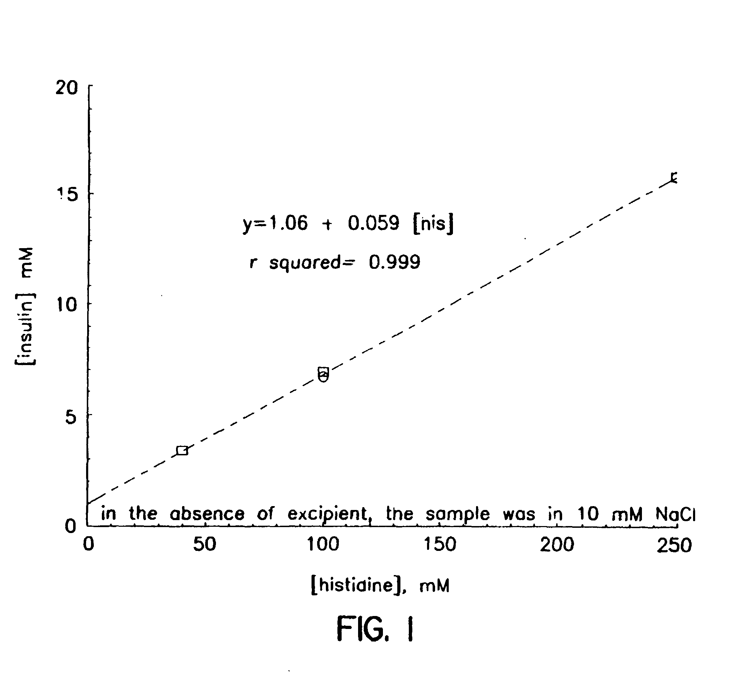

To determine the effect of L-histidine on the solubility limit of 2 zinc/hexamer wild-type human insulin, varying concentrations of insulin were combined with L-histidine. As shown in Figure 1, the use of L-histidine in the buffer solution increased the maximal insulin concentration of an insulin-containing buffer solution. Without L-histidine, at pH 7.5, 10 mM NaCl and at room temperature, the highest concentration of insulin with 2 zinc/hexamer attained was 7.1 mg mL⁻¹ or 1.2 mM. In the presence of 250 mM histidine at its pl, insulin solutions with a concentration as high as 16.5 mM were obtained, representing a 15-fold increase in concentration.

### Example 2

### Effect of L-histidine and L-glycyl-histidine at pl on insulin self-association in the absence of NaCl

To determine the effect of L-histidine and L-glycyl-histidine at their pls on self-association in the absence of NaCl, sedimentation equilibrium studies were performed as described above. As shown in Table I, increased L-histidine concentration resulted in a decrease of the hexamerization equilibrium constant. At pH 7.5, in the absence of any histidine, the sedimentation equilibrium data (collected at 32°C and a rotor speed from 18k to 48k) could be fitted to a dimer hexamer association model with a InK₂₋₆ value of 52.6. The non-ideality coefficient, B, takes into account non-ideality effect arising due to the very low ionic strength of the buffer. Assuming that a single molecular weight species was present, data analysis using the simplest model gave a M_{avg}/M₁ value of 5.5, suggesting that the average size of the insulin aggregates in the absence of any histidine, was slightly less than a hexamer. The observation that 3 insulin dimers assemble to form a hexamer in the presence of zinc at neutral pH is consistent with published data (Brange, *Galenics of insulin,* (1987) Springer Verlag). The sedimentation equilibrium data collected in the presence of 20 to 252 mM histidine at pH 7.6 ± 0.1 at multiple rotor speeds were fitted to a dimer hexamer association model. A decrease in the hexamerization equilibrium constant was observed as the concentration of histidine was increased from 20 to 252 mM. The effect of histidine was observed with insulin at insulin concentrations in the centrifuge cell ranging from as low as 0.01 mM (0.1 mg mL⁻¹) to as high as 13 mM (75 mg mL⁻¹). Despite the fact that histidine dramatically increased the solubility of insulin (solubility limit was 16.5 mM in 250 mM his, pH 7.5 ± 0.1), not all the insulin aggregated under these conditions were dimers and hexamers. Aggregates of multiple hexamers were also found at the highest insulin loading concentration (6 mM) examined in the ultracentrifuge. Only 80% of the insulin initially loaded in the cell remained in solution when the sample reached equilibrium at a rotor speed of 20k rpm. The remaining 20% of the insulin pelleted to the cell bottom as large insoluble aggregates. A rough calculation suggested that the aggregates had an average molecular weight in excess of 100,000, equivalent to about 3 insulin hexamers.

Glycyl-histidine was one of the histidine analogs that was examined for its effect on insulin self-association. At 250 mM, glycyl-histidine also reduced the tendency of insulin to form hexamers but not as effectively as histidine. Analysis of sedimentation equilibrium acquired using interference optics at a rotor speed of 50K using the single species model indicated that the average molecular weight of insulin was that of a tetramer in glycyl-histidine (Table I). As a comparison, at 252 mM histidine (the highest concentration examined in the ultracentrifuge), the average molecular weight of insulin was that of a dimer. Substitution of histidine with another buffer, serinamide, also adjusted to pH 7.5 ± 0.1, failed to reduce the ability of wild-type insulin to self-associate at neutral pH.

**Table I**

| Effect of various buffers on the self-association of wild- type human insulin (with 2 zinc- bound per hexamer) in the absence of sodium chloride at pH 7.5 ± 0.1 and 32°C as analyzed using a dimer-hexamer association model | | | | | |
|---|---|---|---|---|---|
| Buffer | I.S. | M_{avg}/M₁ | InK₂₋₆ | r.m.s. | B |
| no additive¹ | 2.8 | 5.5 | 52.6 | 0.006 | 1.8E-6 |
| serinamide¹ 38 mM | 22 | 5.2 | 25.0 | 0.0080 | 1.7E-6 |
| histidine¹ 20 mM | 3.3 | 4.2 | 20.7 | 0.0080 | 5.5E-6 |
| histidine² 113 mM | 5.6 | 3.3 | 18.3 | 0.0144 | 7.7E-6 |
| histidine³ 240 mM | 6.3 | 2.3 | 14.2 | 0.006 | -0 |
| histidine⁴ 252 mM | 6.3 | 2.6 | 14.4 | 0.0235 | 2.0E-6 |
| gly-his⁵ 250 mM | 27.5 | 4.3 | 18.2 | 0.0464 | 7.9E-7 |
| I.S. is the ionic strength of the buffer in mM | | | | | |
| B (g⁻²L mol) is the non-ideality coefficient, calculated based on an insulin dimer r.m.s. is the root mean square residual between experimental and theoretical data M_{avg}/M, is the average molecular weight of the sedimentating species divided by the molecular weight of the insulin monomer for a single, species non ideal model | | | | | |
| InK₂₋₆ is the natural logarithm of the association constant for the formation of a hexamer from 3 dimers | | | | | |

| | | | | | |
|---|---|---|---|---|---|
| ¹ obtained from uv absorbance scans monitored at 291 nm of a 0.34 mM insulin sample at 18k, 24k, 28k, 34k and 48k rotor speed | | | | | |
| ² obtained from uv absorbance scans monitored at 291 nm of a 0.69 mM insulin sample at 15k, 20k, 25k and 30k rotor speed | | | | | |
| ³ obtained from uv absorbance scans monitored at 291 nm of a 0.35 mM insulin sample at 24k, 28k, 34k and 48k rotor speed | | | | | |
| ⁴ obtained from interference optics of a 6 mM insulin sample at 50k rotor speed | | | | | |
| ⁵ obtained from interference optics of insulin sample at 3 and 6 mM at 50k rotor speed | | | | | |

### Example 3

### Effect of L-histidine and L-glycyl-histidine at pl on the self-association of wild-type human insulin with two zinc bound per hexamer in the presence of NaCl

To determine the effect of L-histidine and L-glycyl-histidine at their pls on insulin self-association in the presence of NaCl, experiments were conducted as described above. Upon increasing the ionic strength of the pH 7.5 samples to about 100 mM with sodium chloride, wild-type human insulin existed mostly as hexamers in the absence of histidine buffer. As shown in Table II, as the concentration of histidine in the insulin samples increased from 50 to 226 mM, there was a modest decrease in the hexamerization equilibrium constant. The effect was not as dramatic as that seen in the absence of salt (see Table I). At 226 mM, the highest histidine concentration examined at an ionic strength of 100 mM, the M_{avg}/M₁ value calculated based on the assumption of a single insulin species, was about 6. In contrast, in the absence of NaCl, insulin was mainly found as dimers in 252 mM histidine, pH 7.6 ± 0.1. In addition, in the presence of 226 mM histidine and 100 mM NaCl, irrespective of the initial loading concentration of insulin, the final concentration of insulin which sedimented with an average size equal or less than that of a dodecamer was about 2 mM. The rest of the insulin sample pelleted to the bottom of the cell at a rotor speed of 20k. Thus, the percent of insulin that formed very large aggregates was considerably higher in histidine buffer containing 100 mM NaCl than without NaCl. In the latter case, the insulin concentration range which resulted in pelleting of large aggregates was about 4.8 mM.

These results show that the effect of histidine on the self-association of insulin is dependent on the ionic milieu of the medium. Preliminary data suggested that the Gibb*s free energy of formation of an insulin dodecamer from 2 hexamers increases as a function of the square root of the ionic strength of the buffer.

In the case of L-glycyl-L-histidine, addition of 50 mM NaCl to the wild-type human insulin sample (raising the total ionic strength to 77.5 mM) gave a hexamerization equilibrium constant close to that observed for insulin in 226 mM histidine and 100 mM NaCl (Table II). Histidine is a zwitterion at its pl, and its effect on insulin seems to be specific. For example, as shown in Table II, the use of taurine, another zwitterion at pH 7.5 ± 0.1, at an ionic strength of about 100 mM, failed to reduce the hexamerization equilibrium constant of insulin.

**Table II**

| Effect of L-histidine and L-glycyl- L-histidine on the self-association of 2 zinc/hexamer wild-type human insulin in the presence of 50 and 100 mM sodium chloride at pH 7.5 ± 0.1 and 32°C as analyzed using a dimer-hexamer association model | | | | |
|---|---|---|---|---|
| **50 mM NaCl** | | | | |
| Buffer | I.S. | M_{avg}/M₁ | InK₂₋₆ | r.m.s. |
| histidine^{1a} 240 mM | 56 | 4.8 | 14.3 | 0.009 |
| gly-his² 247 mM | 77.5 | 4.4 | 16.7 | 0.087 |

| **100 mM NaCl** | | | | |
|---|---|---|---|---|
| Buffer | I.S. | M_{avg}/M₁ | InK₂₋₆ | r.m.s. |
| no additive³ | 103 | 5.8 | 23.9 | 0.007 |
| taurine⁴ 113 mM | 103 | 5.8 | 24.6 | 0.010 |
| histidine⁴ 50 mM | 104 | 4.8 | 21.1 | 0.009 |
| histidine⁵ 113 mM | 106 | 4.5 | 20.7 | 0.012 |
| histidine⁶ 206 mM | 105 | 4.4 | 19.6 | 0.007 |
| histidine^{1b} 226 mM | 106 | 6.4 | 15.6 | 0.009 |

| | | | | |
|---|---|---|---|---|
| ^{1a} obtained from absorbance scans monitored at 295 nm of insulin samples at 3 and 6 mM at 20k and 30k rotor speed | | | | |
| ^{1b} obtained from absorbance scans monitored at 295 nm of insulin samples at 3 and 6 mM at 20k and 30k rotor speed with dimer-hexamer-isodesmic hexamer ideal model with an InK_{6isodesmic} constant of 4.24 | | | | |
| ² obtained from interference optics of insulin samples at 3 and 6 mM at a 50k rotor speed | | | | |
| ³ obtained from uv absorbance scans monitored at 291 nm of an insulin sample at 0.35 mM at 18k, 24k, 34k and 48k rotor speed | | | | |
| ⁴ obtained from uv absorbance scans monitored at 288 nm of a 0.7 mM insulin sample at 15k, 20k, 25k, 30k and 40k rotor speed | | | | |
| ⁵ obtained from uv absorbance scans at 288 (0.35 mM) and 248 nm (0.7 mM) insulin sample at 15k, 20k, 25k, 30k and 40k rotor speed | | | | |
| ⁶ obtained from uv absorbance scans at 248 nm of a 0.35 mM insulin sample at 15k, 20k, 25k and 30k rotor speed | | | | |

InK₆ᵢₛₒ is the natural logarithm of the association constant for the formation of isodesmic hexamers

### Example 4

### Effect of L-histidine at pl on self-association of wild-type Zn-free insulin and a Zn-free. LysPro insulin analog

Sedimentation equilibrium studies were conducted in a XL-I analytical ultracentrifuge at 32°C with native, wild-type human insulin (Zn-free, from Sigma and Humulin® R) as well as a Zn-free Lys^{B28}Pro^{B29} insulin analog (purified from Humalog®, Lilly) as a function of increasing concentration of histidine, pH 7.5, as described above. Interference data were acquired over multiple rotor speeds, pooled and globally analyzed using non linear regression fitted to several models. In the presence of 100 mM NaCl, pH 7.5, the data from wild-type and Lys^{B28}Pro^{B29} human insulin, both free of zinc, can be fitted to a model containing monomer, dimer, hexamer and isodesmic hexamers.

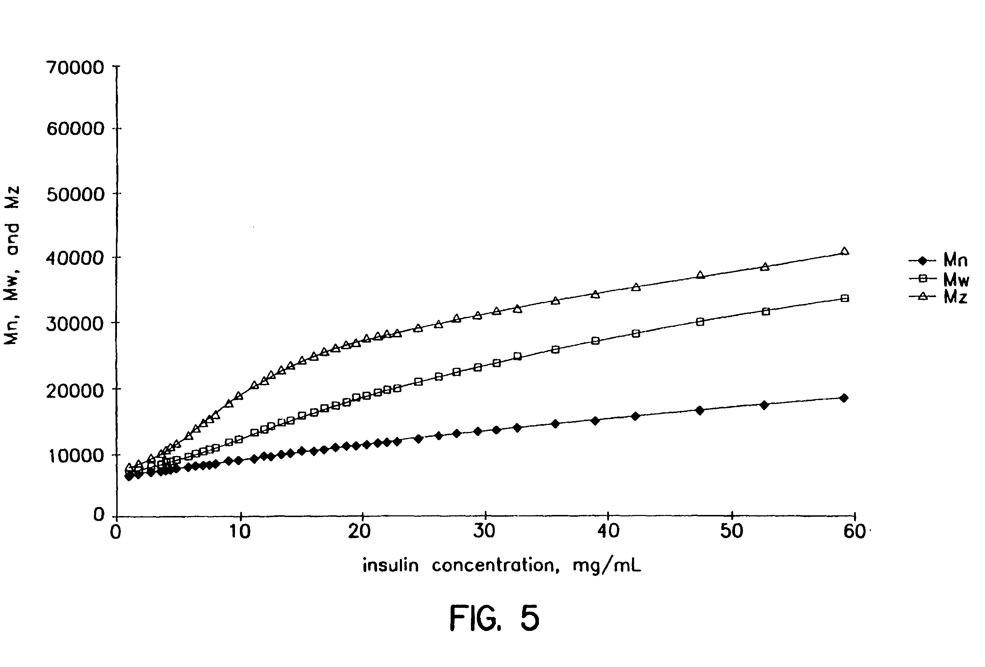

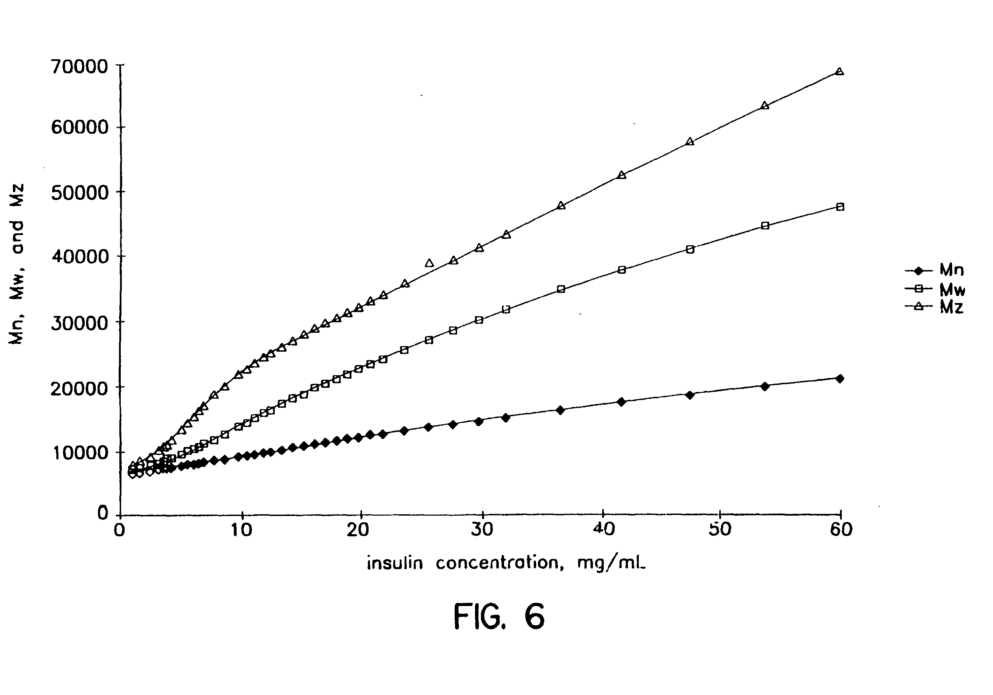

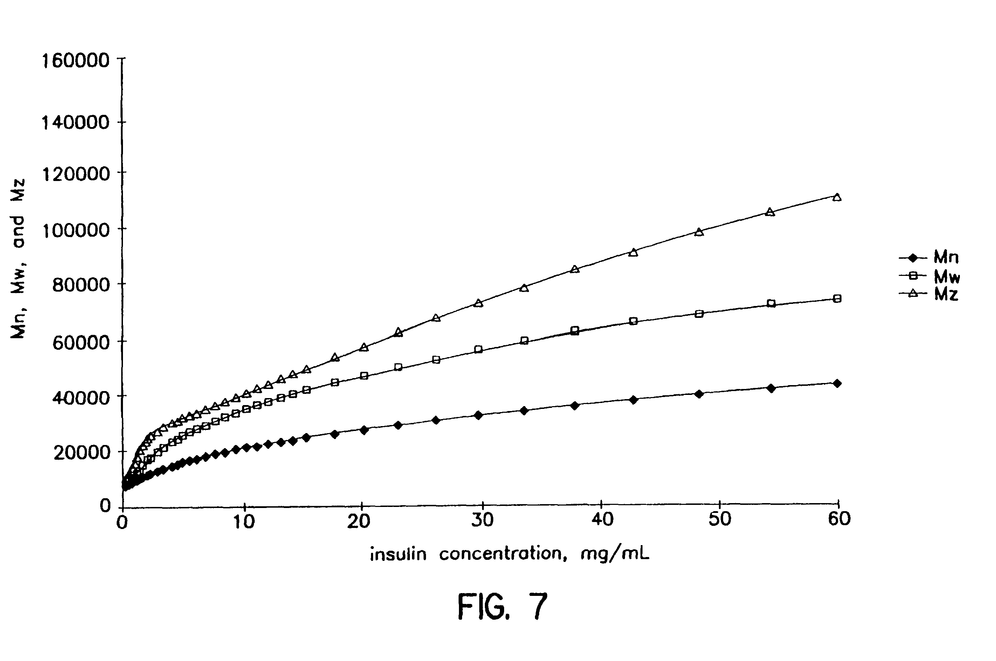

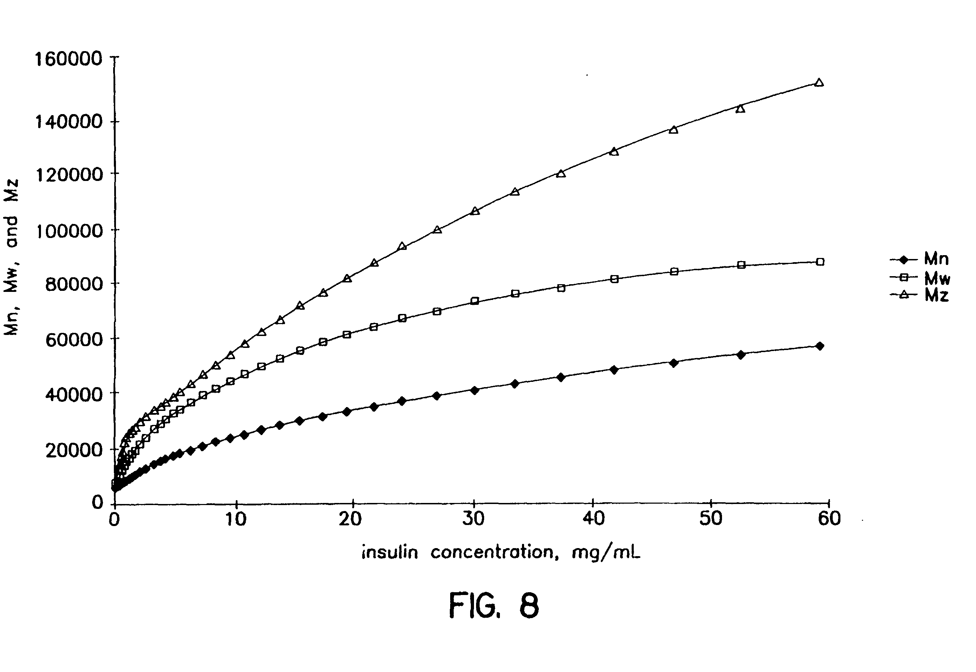

As shown in Table III, for the Zn-free LysPro analog, the value of InK₁₂ did not change significantly with the concentration of histidine but InK₆ᵢₛₒ showed a significant decrease with increasing histidine concentration. The results suggested that histidine had an effect on the self-association properties of the LysPro insulin analog. A succinct method of expressing the degree of aggregation of a protein is to compute an average molecular weight from the association equilibrium constants. Several such averages are well described in the literature (see, e.g., "Analytical Ultracentrifugation in Biochemistry and Polymer Science," Ed. S.E. Hardin, A.J. Rowe, and J.C. Horton, Royal Society of Chemistry, Cambridge, 1992). One, Mn, or the number average molecular weight is defined as cₜₒₜₐₗ/Σ(cᵢ/Mi), where cᵢ is the weight concentration of the i^{th} species with molecular weight Mᵢ. A second type of average, Mw, called the weight average molecular weight, is defined as ΣcᵢMi/cₜₒₜₐₗ. Yet higher molecular weight averages such as M_{z} may be defined, where M_{z}=ΣcᵢMᵢ²/ΣcᵢMᵢ. Figure 5 shows the concentration dependence of these three averages for the LysPro insulin analog in the presence of 240 mM histidine (calculated from InK₁₂, InK₂₆ and InK₆ᵢₛₒ values for 240 mM histidine in Table III) while Figure 6 shows a similar plot for the situation in the absence of histidine (calculated from InK values for 0 mM histidine in Table III). Figure 7 shows the similar plot for zinc-free wild-type insulin in the presence of 240 mM histidine (calculated from InK₁₂, InK₂₆ and InK₆ᵢₛₒ values for 240 mM histidine in Table IV) while Figure 8 shows a comparable plot in the absence of histidine (calculated from InK values for 0 mM histidine in Table IV).

It is evident from these plots that the presence of histidine substantially reduces the average molecular weight of the insulin, and that this effect is more pronounced for the LysPro analog.

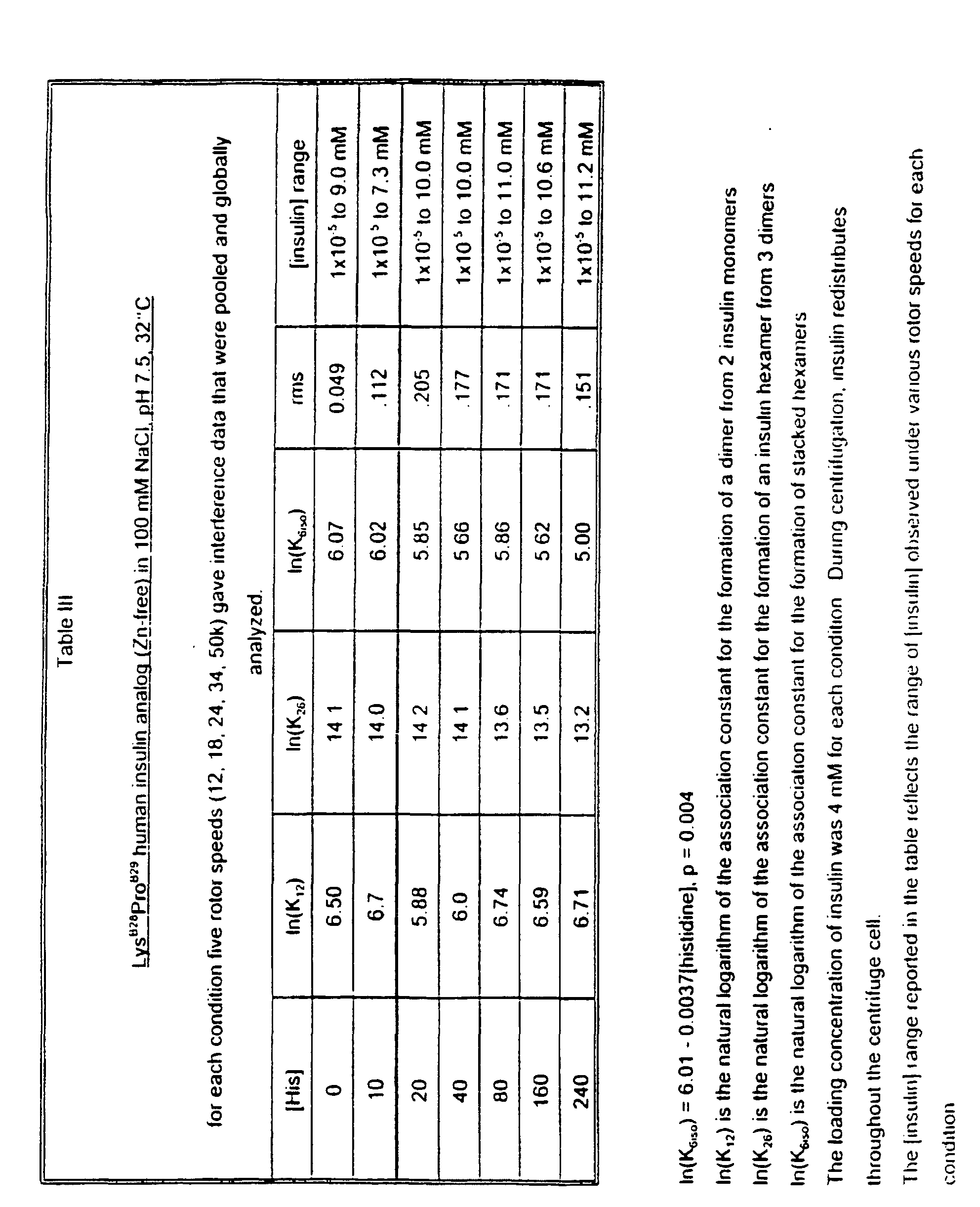

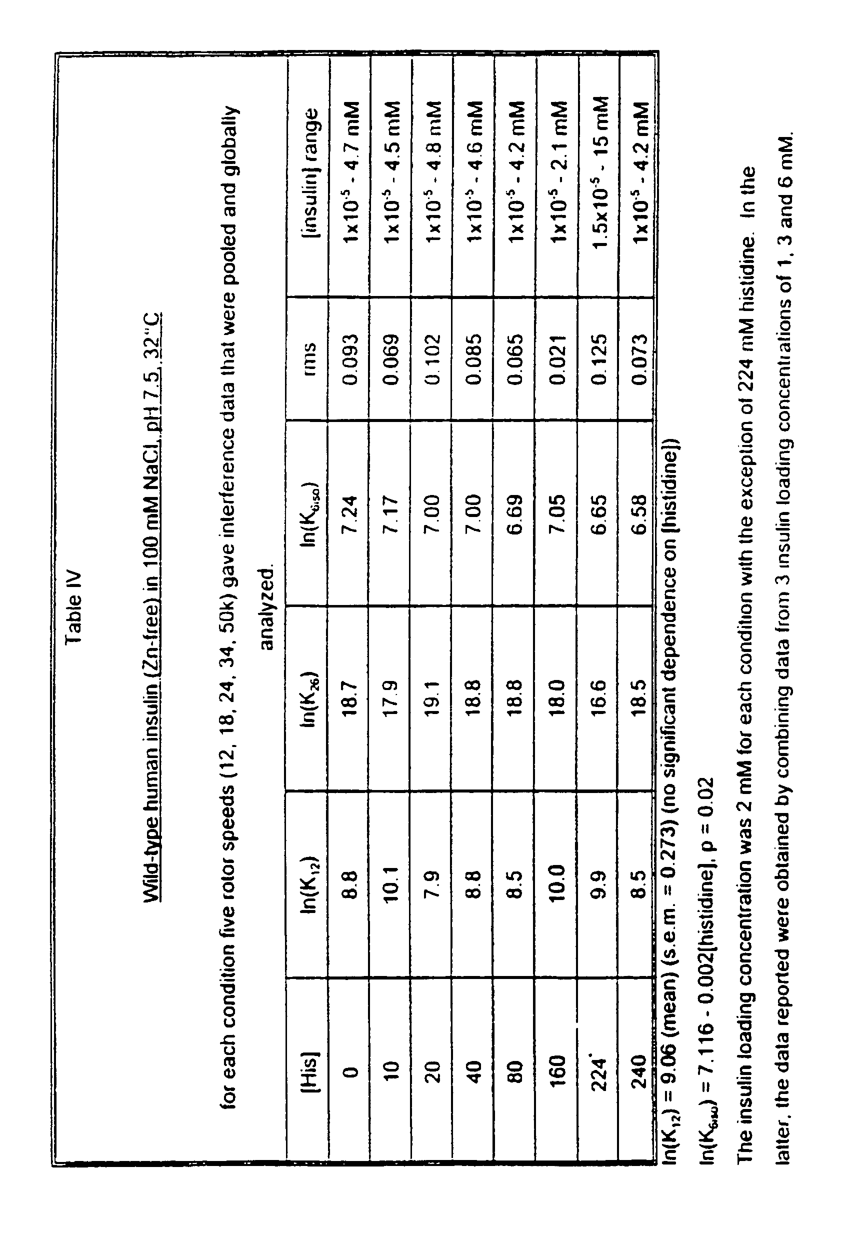

### Example 5

### Effect of L-histidine at its pi on the self-association of other proteins

The effect of L-histidine on self-association of lysozyme and β-lactoglobulin was also studied, using the methods described above. Lysozyme and β-lactoglobulin are well-studied proteins which predominately exist in a monomer-dimer equilibrium at alkaline pH (Kim et al., *Chemical Reviews* (1977) 77:659-690). Lysozyme sedimentation equilibrium data at alkaline pH are better modeled by a monomer-dimer-tetramer system than by a more simple monomer-dimer system (Holladay, Ph.D. Dissertation (1973) Emory University). The self-association behavior of lysozyme without L-histidine at 4°C was assessed by globally analyzing interference fringe data from 14k, 18k, and 30k rpm. The results are given in Table V.

The expected noise in fringe measurement from the Beckman XL-I system is about 0.02 to 0.04 fringes. The 4°C data are best described by an ideal monomer-dimer-tetramer (1-2-4) system. Note that any model which contains aggregates above the dimer size yields essentially identical estimates for the In(K₁₂). Since these results (presented below) on the effect of L-histidine on lysozyme dimerization do not tend to be model-dependent, modeling was done with an ideal 1-2-4 system. Inclusion of a second virial coefficient failed to significantly lower the r.m.s. residual. Isodesmic type I has all aggregates present with identical association constants. Type II has only even aggregates present Type III has a dimerization constant different from subsequent association steps which are presumed to be isodesmic. Type IV has only even aggregates presumed to be present with a dimerization constant different from subsequent association steps which are presumed to be isodesmic. The equations for the isodesmic models are known in the art, and have been described (Tang et al., *Biophys. Chem.* (1977) 7:121-139). Note that for the isodesmic model IV that the In(K₁₄) is 17.9, close to that estimated from the 1-2-4 model. The predicted amounts of aggregates beyond tetramer are quite small for all the isodesmic models.

The effect of L-histidine on lysozyme dimerization is given in Table VI. The results in Table VI were generated using an ideal 1-2-4 model (monomer-dimer-tetramer) and global analysis of two rotor speeds differing by 4k rpm. The effect of L-histidine on β-lactoglobulin dimerization at three temperatures is given in Table VII. For both proteins, there appear to be modest decreases in the dimerization equilibrium constant with increasing temperature. For this analysis, it was implicitly assumed that the effective buoyant molecular weight of any aggregate in the presence of L-histidine is an integer multiple of the effective buoyant molecular weight of the monomer. This implies that the B_{AM} term of any aggregate is an integer multiple of the B_{AM} term for the monomer. Since in reality the overall shape of an aggregate is likely to be somewhat different than that of the monomer, it is possible that the modest decreases in the dimerization constants for lysozyme and β-lactoglobulin may reflect the failure of the assumption that the buoyant molecular weight of the aggregate is an integer multiple of that of the monomer. However it must be noted that the returned value of In(K₁₂) does not tend to be sensitive to changes of a few percent in the monomer buoyant molecular weight.

**Table V**

| Effect of the choice of self-association model on the In(K₁₂) of lysozyme in 150 mM NaCl. pH 7.6. 4°C. from a global analysis of interference data obtained at 14k. 18k and 30k rotor speeds | | | |
|---|---|---|---|
| model | In(K₁₂) | In(K_{additional}) | r.m.s. residual |
| 1-2 | 6.728 | n.a. | 0.114 |
| 1-2-4 | 5.431 | 17.9(1-4) | 0.036 |
| non ideal 1-2-4 | 5.436 | 18.0 | 0.036 |
| isodesmic Type I | 5.552 | n.a. | 0.048 |
| isodesmic Type II | 5.582 | n.a. | 0.054 |
| isodesmic Type III | 5.247 | 5.716 | 0.037 |
| isodesmic Type IV | 5.577 | 5.966 | 0.041 |
| 1-2 denotes model in which monomer exists in equilibrium with dimer 1-2-4 denotes model in which monomer exists in equilibrium with dimer and tetramer | | | |
| InK₁₋₂ is the equilibrium constant for formation of a dimer from 2 monomers | | | |
| InK₁₋₄ is the equilibrium constant for the formation of a tetramer from 4 monomers | | | |

**Table Vl**

| Effect of L-histidine on self-association of lysozyme at pH 7.6. 150 mM NaCl at multiple rotor speeds using 3 mm centerpiece | | | |
|---|---|---|---|
| | | InK¹ | InK¹ |
| Buffer | | 14k & 18k, 4°C | 16k & 20k. 20°C |
| no additive | 1-2 | 5.64 (0.06)² | 5.15 (0.05) |
| | 1-4 | 17.97 (0.03) | 16.10 (0.06) |
| | r.m.s. | 0.039 | 0.043 |
| | | | |
| 100 mM his | 1-2 | 4.85 (0.04) | 4.32 (0.05) |
| | 1-4 | 17.08 (0.01) | 15.84 (0.02) |
| | r.m.s. | 0.032 | 0.040 |
| | | | |
| 250 mM his | 1-2 | 4.13 (0.05) | 3.48 (0.05) |
| | 1-4 | 16.24 (0.01) | 15.32 (0.01) |
| | r.m.s. | 0.032 | 0.031 |

| | | | |
|---|---|---|---|
| ¹ obtained from fitting interference data globally to a model containing monomer-dimer-tetramer existing in equilibrium. The InK₁₋₂ and InK₁₋₄ are the equilibrium constants for the formation of a dimer and tetramer, respectively, from a monomer. | | | |
| ² values in parentheses are bootstrap standard error of InK | | | |

**Table VII**

| Effect of L-histidine on the self-association of β lactoglobulin at pH 7.6. 150 mM NaCl at multiple rotor speeds using a 3 mm centerpiece | | | |
|---|---|---|---|
| | 4°C | 20°C | 32°C |
| | InK¹ | InK¹ | InK¹ |
| Buffer | 14k & 18K | 16k & 20K | 22k & 26K |
| no additive | 11.34(0.12) [0.008] | 10.26(0.12) [0.010] | 9.28(0.03) [0.008] |
| | | | |
| 250 mM his | 9.52(0.06) [0.005] | 8.48(0.02) [0.009] | 8.03(0.02) [0.005] |

| | | | |
|---|---|---|---|
| ¹ obtained from fitting absorbance data globally to an ideal monomer-dimer model with floating baseline offset. Bootstrap standard errors of InK values are given in parentheses. Figures in square parentheses denotes root mean squared residuals. | | | |

Based on the above experiments, it is evident that histidine and histidine analogs are able to decrease self-association of insulin and insulin analogs, with and without zinc. In the case of wild-type insulin, containing 2 zinc molecules/hexamer, over the concentration range examined, the sedimentation equilibrium data at pH 7.5 can be fitted to a non-ideal dimer-hexamer model in the absence of NaCl or to an ideal dimer-hexamer model (when the loading insulin concentration is below 1 mM) in the presence of 100 mM NaCl. Moreover, there is a striking effect of histidine concentration on the InK₂₆ value of native human 2 zinc/hexamer insulin whether NaCl is present or not. In the absence of zinc, the effect of histidine on the LysPro analog is more pronounced than the wild-type insulin. Histidine is also able to decrease self-association of other totally unrelated proteins, such as lysozyme and β-lactoglobulin.

Thus, methods for decreasing self-association and increasing solubility of polypeptide agents are disclosed.

## Claims

1. Use of a polypeptide and a histidine compound in an amount sufficient to decrease the tendency of said polypeptide to self-associate in the manufacture of a composition for delivering said polypeptide through the skin by electrotransport or passive transdermal delivery.

2. The use of claim 1, wherein the histidine compound is L-histidine or L-glycyl-histidine.

3. The use of any of claims 1 or 2, wherein the polypeptide is an insulin compound.

4. The use of claim 3, wherein the insulin compound is a human insulin compound.

5. The use of claim 4, wherein the insulin compound is a zinc-free human insulin compound.

6. The use of claim 4 or 5, wherein the insulin compound is a human Lys^{B28}Pro^{B29} insulin analog.

7. The use of any one of claims 3 to 6, wherein insulin hexamer formation is decreased.

8. The use of any one of claims 1 to 7, wherein the concentration of the histidine compound is at least about 10 mmolar.

9. The use of any one of claims 1 to 8, wherein the pH of the composition is pH 7 to pH 8.

10. Use as claimed in claim 1 for delivering an insulin compound through the skin by electrotransport wherein the molar ratio of the insulin compound to the histidine compound is 1:10 to 1:1000.

11. A composition suitable for delivery through a body surface by electrotransport or passive transdermal delivery comprising an insulin compound and a histidine compound in an amount sufficient to decrease the tendency of said insulin compound to self-associate.

12. An electrotransport delivery device or passive transdermal delivery device comprising a composition as defined in any one of claims 1 to 10.

## Patentansprüche

1. Verwendung eines Polypeptids und einer Histidinverbindung in einer Menge, die ausreicht, die Neigung des Polypeptids zur Selbstassoziation zu verringern, zur Herstellung einer Zusammensetzung zur Verabreichung des Polypeptids durch die Haut mittels Elektrotransport oder passive transdermale Verabreichung.

2. Verwendung nach Anspruch 1, wobei es sich bei der Histidinverbindung um L-Histidin oder L-Glycyl-Histidin handelt.

3. Verwendung nach einem der Ansprüche 1 oder 2, wobei das Polypeptid eine Insulinverbindung ist.

4. Verwendung nach Anspruch 3, wobei es sich bei der Insulinverbindung um eine Humaninsulinverbindung handelt.

5. Verwendung nach Anspruch 4, wobei es sich bei der Insulinverbindung um eine zinkfreie Humaninsulinverbindung handelt.

6. Verwendung nach Anspruch 4 oder 5, wobei die Insulinverbindung ein Human-Lys^{B28}Pro^{B29}-Insulinanalogon ist.

7. Verwendung nach einem der Ansprüche 3 bis 6, wobei die Insulinhexamerbildung verringert ist.

8. Verwendung nach einem der Ansprüche 1 bis 7, wobei die Konzentration der Histidinverbindung wenigstens etwa 10 mmolar ist.

9. Verwendung nach einem der Ansprüche 1 bis 8, wobei der pH der Zusammensetzung etwa pH 7 bis pH 8 ist.

10. Verwendung nach Anspruch 1 zur Verabreichung einer Insulinverbindung durch die Haut mittels Elektrotransport, wobei das Molverhältnis der Insulinverbindung zu der Histidinverbindung 1:10 bis 1:1000 beträgt.

11. Zusammensetzung, die zur Verabreichung durch eine Körperoberfläche mittels Elektrotransport oder passive transdermale Verabreichung geeignet ist, umfassend eine Insulinverbindung und eine Histidinverbindung in einer Menge, die ausreicht, die Neigung der Insulinverbindung zur Selbstassoziation zu verringern.

12. Vorrichtung zur Verabreichung mittels Elektrotransport oder Vorrichtung zur passiven transdermalen Verabreichung, umfassend eine Zusammensetzung wie in einem der Ansprüche 1 bis 10 definiert.

## Revendications

1. Utilisation d'un polypeptide et d'un composé d'histidine en une quantité suffisante pour diminuer la tendance du polypeptide à s'autoassocier dans la fabrication d'une composition pour l'administration du polypeptide à travers la peau par électrotransport ou par administration transdermique passive.

2. Utilisation suivant la revendication 1, dans laquelle le composé d'histidine est la L-histidine ou la L-glycyl-histidine.

3. Utilisation suivant l'une quelconque des revendications 1 ou 2, dans laquelle le polypeptide est un composé d'insuline.

4. Utilisation suivant la revendication 3, dans laquelle le composé d'insuline est un composé d'insuline humain.

5. Utilisation suivant la revendication 4, dans laquelle le composé d'insuline est un composé d'insuline humain exempt de zinc.

6. Utilisation suivant la revendication 4 ou 5, dans laquelle le composé d'insuline est un analogue d'insuline humain Lys^{B28} Pro^{B29}.

7. Utilisation suivant l'une quelconque des revendications 3 à 6, dans laquelle la formation d'insuline hexamère est diminuée.

8. Utilisation suivant l'une quelconque des revendications 1 à 7, dans laquelle la concentration du composé d'histidine est d'au moins 10 mmolaire environ.

9. Utilisation suivant l'une quelconque des revendications 1 à 8, dans laquelle le pH de la composition va de pH 7 à pH 8.

10. Utilisation suivant la revendication 1, pour administrer un composé d'insuline à travers la peau par électrotransport, dans laquelle le rapport molaire du composé d'insuline au composé d'histidine est compris entre 1:10 et 1:1000.

11. Composition propre à l'administration à travers une surface du corps par électrotransport ou par administration transdermique passive, comprenant un composé d'insuline et un composé d'histidine en une quantité suffisante pour diminuer la tendance du composé d'insuline à s'autoassocier.

12. Dispositif d'administration par électrotransport ou d'administration transdermique passive, comprenant une composition telle que définie suivant l'une quelconque des revendications 1 à 10.
